# DEMANDE DE BREVET EUROPEEN

(11) **EP 3 708 141 A1**
(43) Date de publication de la demande: **16.09.2020**
(21) Numéro de dépôt: 20167688.9
(22) Date de dépôt: 20.06.2013
(51) Int. Cl.: A61K 8/02, A61K 8/25, A61K 8/81, A61Q 5/02

(54) **PROCÉDÉ DE TRAITEMENT CAPILLAIRE AVEC UNE COMPOSITION COSMÉTIQUE COMPRE-NANT DES PARTICULES D'AÉROGEL DE SILICE HYDROPHOBE ET UN POLYMÈRE ÉPAISSIS-SANT ACRYLIQUE PARTICULIER**

(30) Priorité: 21.06.2012 FR 1255855; 20.11.2012 US 201261728265 P
(62) Demande divisionnaire de: 13737336.1
(71) Demandeur: L'Oréal SA, 75008 Paris (FR)
(72) Inventeur: KHENNICHE, Samira, 93400 SAINT-OUEN (FR); PLOS, Grégory, 94152 CHEVILLY LARUE (FR)
(74) Mandataire: L'Oreal

(57) **Abrégé**

La présente invention concerne un procédé de traitement capillaire permettant en particulier de retarder voire d'éviter le regraissage des cheveux et/ou du cuir chevelu, comprenant l'application d'une composition cosmétique comprenant des particules d'aérogel de silice hydrophobe et au moins un polymère épaississant acrylique particulier.

## Description

La présente invention concerne un procédé de traitement capillaire avec une composition cosmétique comprenant des particules d'aérogel de silice hydrophobe et un polymère épaississant acrylique particulier.

Les chevelures ont tendance à perdre certaines de leurs qualités sous l'action de facteurs tels que le regraissage naturel, la sueur, l'élimination de squames, la pollution, l'humidité et autres facteurs. L'aspect visuel ou le toucher des cheveux peut ainsi être dégradé. Le regraissage, par exemple dû à la pollution, alourdit les cheveux qui ont alors tendance à se mettre en paquets. Les cheveux peuvent être difficiles à coiffer en plus d'avoir par exemple une brillance grasse ou un toucher ciré désagréable. Pour lutter contre ces désagréments, on peut utiliser des compositions détergentes, par exemple des shampoings, afin d'éliminer les salissures (sébum, sueur, pollution, etc.) ou les pellicules, et détendre les cheveux. La chevelure est ensuite rincée puis séchée. Les shampooings doivent être renouvelés régulièrement, par exemple au bout de quelques jours voire au bout de quelques heures. Toutefois, les shampooings, à base de quantités importantes de tensioactifs, peuvent générer des désagréments tels que des picotements sur le cuir chevelu ou dans les yeux.

Les compositions de shampoing ou de lavage de la peau peuvent également associer aux tensioactifs, des absorbeurs de sébum pour permettre de prolonger dans le temps la perception de propreté des cheveux ou de la peau. Toutefois, on a constaté que l'efficacité de ces produits est encore insuffisante par rapport aux attentes des consommateurs. En effet, ils ne permettent pas de réduire de manière significative la fréquence de lavage des cheveux notamment, fréquence qui peut différer selon le pays ou même de la région concernée et qui peut aller de un à deux shampooings par jour à un ou deux shampooings par semaine.

A ce jour, aucune composition cosmétique d'hygiène, notamment capillaire, ne permet de ralentir de manière significative le regraissage, notamment des cheveux.

La présente invention a pour but de proposer des compositions cosmétiques palliant ces inconvénients.
Les compositions selon l'invention permettent de conserver une perception de cheveux propres pendant un temps plus long qu'avec un shampoing usuel; à titre d'exemple, cette perception de cheveux propres peut être d'une semaine pour les personnes se lavant habituellement les cheveux 2 à 3 fois par semaine, et peut être d'au moins trois jours pour les personnes se lavant habituellement les cheveux tous les jours.
Par ailleurs, les compositions selon l'invention permettent d'obtenir des performances de nettoyage au moins identiques à celles d'un shampooing standard, notamment un très bon pouvoir de détergence.

L'invention a donc pour objet un procédé de traitement capillaire, pour le soin, le traitement cosmétique et/ou le nettoyage des cheveux et/ou du cuir chevelu, en particulier permettant de retarder voire d'éviter le regraissage des cheveux et/ou du cuir chevelu, comprenant l'application d'une composition cosmétique comprenant des particules d'aérogel de silice hydrophobe et au moins un polymère épaississant acrylique choisi parmi les homopolymères d'acide (méth)acrylamido alkyl(C1 C4) sulfonique, éventuellement réticulés.

On a constaté que l'utilisation des compositions selon l'invention permet de réduire de manière significative le regraissage des cheveux et/ou du cuir chevelu, et permet ainsi de réduire la fréquence de lavage.
En outre, la composition selon l'invention permet d'obtenir de bonnes propriétés de conditionnement des cheveux, notamment en terme de douceur, souplesse, lissage et démêlage, tout en ayant une répartition et un étalement sur la chevelure améliorés.

Dans la présente description, l'expression "au moins un" est équivalente à l'expression "un ou plusieurs" et peut y être substituée.
Dans la présente description, l'expression "compris entre" est équivalente à l'expression "allant de " et peut y être substituée.

### Particules d'aérogel de silice hydrophobe

La composition selon l'invention comprend donc des particules d'aérogel de silice hydrophobe.

Les aérogels sont des matériaux poreux ultra légers, dont les premiers ont été réalisés par Kristler en 1932.
Ils sont généralement synthétisés par un procédé sol-gel en milieu liquide puis séchés par extraction d'un fluide supercritique. Le fluide supercritique le plus communément utilisé est le CO₂ supercritique. Ce type de séchage permet d'éviter la contraction des pores et du matériau. D'autres types de séchage permettent également d'obtenir des matériaux poreux à partir de gel, à savoir par exemple (i) le séchage par cryodessiccation, consistant à solidifier à faible température le gel puis à sublimer le solvant et (ii) le séchage par évaporation. Les matériaux ainsi obtenus sont appelés respectivement cryogels et xérogels. Le procédé sol-gel et les différents séchages sont décrits en détail dans Brinker CJ., and Scherer G.W., Sol-Gel Science: New York: Academic Press, 1990.

Par "silice hydrophobe", on entend toute silice dont la surface est traitée par des agents de silylation, par exemple par des silanes halogénés tels que des alkylchlo-rosilanes; des siloxanes en particulier des diméthylsiloxanes tels que l'hexaméthyldisiloxane; ou des silazanes, de manière à fonctionnaliser les groupements OH par des groupements silyles Si-Rn, par exemple des groupements triméthylsilyles.

De préférence, les particules d'aérogel de silice hydrophobe susceptibles d'être utilisées dans la présente invention présentent avantageusement une surface spécifique par unité de masse (SM) allant de 500 à 1500 m²/g, de préférence de 600 à 1200 m²/g et mieux de 600 à 800 m²/g.
De préférence, les particules d'aérogel de silice hydrophobe susceptibles d'être utilisées dans la présente invention présentent avantageusement une capacité d'absorption d'huile mesurée au WET POINT allant de 5 à 18 ml/g de particules, de préférence de 6 à 15 ml/g et mieux de 8 à 12 ml/g.
De préférence, les particules d'aérogel de silice hydrophobe susceptibles d'être utilisées dans la présente invention présentent avantageusement une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.
De préférence, les particules d'aérogel de silice hydrophobe susceptibles d'être utilisées dans la présente invention présentent avantageusement une densité tassée p allant de 0,04 g/cm³ à 0,10 g/cm³, de préférence de 0,05 g/cm³ à 0,08 g/cm³.
De préférence, les particules d'aérogel de silice hydrophobe susceptibles d'être utilisées dans la présente invention présentent avantageusement une surface spécifique par unité de volume (SV) allant de 5 à 60 m²/cm³, de préférence de 10 à 50 m²/cm³ et mieux de 15 à 40 m²/cm³.

Selon un mode de réalisation préféré, les particules d'aérogel de silice hydrophobe selon l'invention présentent une surface spécifique par unité de masse (SM) allant de 500 à 1500 m²/g, de préférence de 600 à 1200 m²/g et mieux de 600 à 800 m²/g, et une taille exprimée en diamètre moyen (D[0,5]) allant de 1 à 30 µm et/ou une capacité d'absorption d'huile mesurée au WET POINT allant de 5 à 18 ml/g de particules, de préférence de 6 à 15 ml/g et mieux de 8 à 12 ml/g.

Selon un autre mode de réalisation préféré, les particules d'aérogel de silice hydrophobe utilisées dans la présente invention présentent une surface spécifique par unité de masse (SM) allant de 600 à 800 m²/g et une taille exprimée en diamètre moyen en volume (D[0,5]) allant de 5 à 20 µm, mieux de 5 à 15 µm.

La surface spécifique par unité de masse peut être déterminée par la méthode d'absorption d'azote appelée méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.
La capacité d'absorption mesurée au WET POINT, et notée Wp, correspond à la quantité d'huile qu'il faut additionner à 100 g de particules pour obtenir une pâte homogène. Elle est mesurée selon la méthode dite de Wet Point ou méthode de détermination de prise d'huile de poudre selon le principe décrit dans la norme NF T 30-022. Elle correspond à la quantité d'huile adsorbée sur la surface disponible de la poudre et/ou absorbée par la poudre par mesure du Wet Point, décrite ci-dessous :
On place une quantité m = 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.
La prise d'huile (capacité d'absorption d'huile) correspond au rapport Vs / m.
Les tailles des particules d'aérogel selon l'invention peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.
Dans le cadre de la présente invention, la densité tassée peut être appréciée selon le protocole suivant, dit protocole de la densité tassée : 40 g de poudre sont versés dans une éprouvette graduée puis l'éprouvette est placée sur un appareil STAV 2003 de chez STAMPF VOLUMETER. L'éprouvette est ensuite soumise à une série de 2500 tassements (cette opération est recommencée jusqu'à ce que la différence de volume entre 2 essais consécutifs soit inférieure à 2 %); puis le volume final Vf de poudre tassée est mesuré directement sur l'éprouvette. La densité tassée est déterminée par le rapport masse(m)/Vf, en l'occurrence 40/Vf (Vf étant exprimé en cm³ et m en g).
La surface spécifique par unité de volume est donnée par la relation : SV = SM x p, où p est la densité tassée exprimée en g/cm³ et SM est la surface spécifique par unité de masse exprimée en m²/g, telles que définies plus haut.

Les particules d'aérogel de silice hydrophobe utilisées selon la présente invention sont de préférence des particules d'aérogel de silice silylée (nom INCI silica silylate).
La préparation de particules d'aérogels de silice hydrophobe modifiées en surface par silylation est décrite plus avant dans le document US 7,470,725.
On utilisera en particulier des particules d'aérogels de silice hydrophobe modifiée en surface par groupements triméthylsilyles.

A titre de d'aérogels de silice hydrophobe utilisables dans l'invention, on peut citer par exemple l'aérogel commercialisé sous la dénomination VM-2260 (nom INCI Silica silylate), par la société Dow Corning, dont les particules présentent une taille moyenne d'environ 1000 microns et une surface spécifique par unité de masse allant de 600 à 800 m²/g.
On peut également citer les aérogels commercialisés par la société Cabot sous les références AEROGEL TLD 201, AEROGEL OGD 201 et AEROGEL TLD 203, ENOVA AEROGEL MT 1100, ENOVA AEROGEL MT 1200.
On utilisera plus particulièrement l'aérogel commercialisé sous la dénomination VM-2270 (nom INCI Silica silylate), par la société Dow Corning, dont les particules présentent une taille moyenne allant de 5 à 15 microns et une surface spécifique par unité de masse allant de 600 à 800 m²/g.

On peut bien évidemment utiliser un mélange de particules d'aérogel de silice hydrophobe.

Les compositions selon l'invention peuvent comprendre les particules d'aérogel de silice hydrophobe en une quantité comprise entre 0,01 et 20% en poids, de préférence entre 0,05 et 10% en poids, préférentiellement entre 0,1 et 5% en poids par rapport au poids total de la composition.

### Polymères épaississants

La composition selon l'invention comprend également au moins un polymère épaississant acrylique. On peut bien évidemment utiliser un mélange de polymères épaississants acryliques.

Par polymère acrylique, on entend au sens de la présente invention un polymère résultant d'une réaction de polymérisation mettant en œuvre un ou plusieurs monomère(s) de structure: 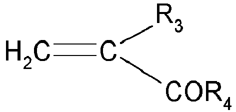
avec R3 désignant un atome d'hydrogène ou un radical alkyl en C1-C4, linéaire ou ramifié,
et R4 désignant un atome d'hydrogène, un radical alkyl en C1-C4 linéaire ou ramifié, un radical NR5R6 ou un radical alcoxy en C1-C30 linéaire ou ramifié, lesdits radicaux étant éventuellement substitués par un ou plusieurs OH et/ou radical ammonium quaternaire;
R5 et R6 désignant indépendamment l'un de l'autre, un atome d'hydrogène ou un radical alkyl, linéaire ou ramifié, en C1-C30 éventuellement oxyalkyléné, comportant éventuellement un groupement sulfonique.

De préférence R3 désigne un atome d'hydrogène ou un radical méthyle.

Par polymère épaississant, on entend au sens de la présente invention un polymère présentant en solution ou en dispersion à 1% en poids de matière active dans l'eau ou dans l'éthanol, à 25°C, une viscosité supérieure à 0,2 Poise, à un taux de cisaillement de 1 s⁻¹. La viscosité est mesurée avec un viscosimètre HAAKE RS600 de THERMO ELECTRON. Ce viscosimètre est un viscosimètre à contrainte imposée en géométrie cône plan (par exemple de diamètre 60 mm)

Les polymères épaississants acryliques susceptibles d'être utilisés dans la présente invention sont choisis parmi les homopolymères d'acide (méth)acrylamido alkyl(C1-C4) sulfonique, éventuellement réticulés.

Les homopolymères d'acide (méth)acrylamido alkyl(C1-C4) sulfonique sont de préférence réticulés. Plus particulièrement, ils sont partiellement ou totalement neutralisés. Ce sont des polymères hydrosolubles ou gonflables dans l'eau. On peut citer notamment l'acide polyacrylamido-méthane-sulfonique, l'acide polyacrylamido-éthane-sulfonique, l'acide polyacrylamido-propane-sulfonique, l'acide poly-2-acrylamido-2-méthylpropane-sulfonique, l'acide poly-2-méthacrylamido-2-méthylpropane-sulfonique et l'acide poly-2-acrylamido-n-butane-sulfonique.

Les homopolymères d'acide poly-2-acrylamido-2-méthylpropane-sulfonique réticulés et partiellement ou totalement neutralisés sont connus, notamment par la demande de brevet DE-19625810.
Ils sont en général caractérisés par le fait qu'ils comprennent, distribués de façon aléatoire :
a) de 90 à 99,9% en poids de motifs de formule (I) suivante : 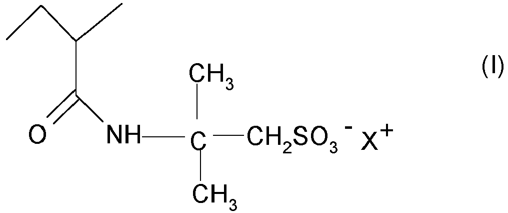 dans laquelle X⁺ désigne un cation ou un mélange de cations, dont H+ ,
b) de 0,01 à 10% en poids d'au moins un motif réticulant ayant au moins deux doubles-liaisons oléfiniques,
les proportions en poids étant définies par rapport au poids total du polymère;
X⁺ représente un cation ou un mélange de cations choisis en particulier parmi un proton, un cation de métal alcalin, un cation équivalent de celui d'un métal alcalino-terreux ou l'ion ammonium.
De préférence, l'acide poly-2-acrylamido-2-méthylpropane-sulfonique réticulé et neutralisé comporte de 98 à 99,5 % en poids de motifs de formule (I) et de 0,5 à 2 % en poids de motifs réticulants.

Les motifs réticulants ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétra-allyl-oxyéthane ou d'autres allyl- ou vinyl-éthers d'alcools polyfonctionnels, le diacrylate de tétraéthylèneglycol, la triallylamine, le triméthylolpropane-diallyléther, le méthylène-bis-acrylamide ou le divinylbenzène.
Les motifs réticulants ayant au moins deux doubles-liaisons oléfiniques sont plus particulièrement encore choisis parmi ceux répondant à la formule générale (II) suivante : 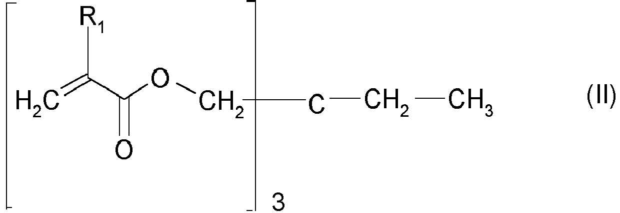 dans laquelle R₁ désigne un atome d'hydrogène ou un alkyle en C₁-C₄ et plus particulièrement méthyle (triméthylol propane triacrylate).

Les acides poly-2-acrylamido-2-méthylpropane-sulfoniques réticulés et partiellement ou totalement neutralisés sont généralement connus sous les appellations "Ammonium Polycrylamido-2-methylpropanesulfonate" ou encore "Ammonium Polyacryldimethyltauramide" (appellation I.N.C.I.).
Un produit particulièrement préféré selon l'invention est celui vendu par la société CLARIANT sous la dénomination commerciale HOSTACERIN AMPS; c'est un acide poly-2-acrylamido-2-méthylpropane sulfonique réticulé et partiellement neutralisé par l'ammoniaque.

La composition selon l'invention comprend le ou les polymères épaississants acryliques en une quantité de préférence comprise entre 0,01 et 2% en poids, notamment 0,1 à 0,5% en poids, par rapport au poids total de la composition.

### Autres ingrédients

La composition cosmétique selon l'invention comprend généralement un milieu cosmétiquement acceptable, c'est-à-dire un milieu compatible avec les matières kératiniques telles que la peau du visage ou du corps, les lèvres, les cheveux, les cils, les sourcils et les ongles.

Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.
Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

La composition selon l'invention peut être aqueuse ou anhydre. Elle est de préférence aqueuse et comprend alors de l'eau à une concentration allant de préférence de 5 à 98% en poids, notamment de 20 à 95% en poids, mieux de 50 à 90% en poids, par rapport au poids total de la composition
La composition peut également comprendre un ou plusieurs solvants organiques liquides à 25°C, 1 atm., notamment hydrosolubles, tels que les alcools en C1-C7; on peut notamment citer les monoalcools aliphatiques ou aromatiques en C1-C7, les polyols et les éthers de polyols en C3-C7, qui peuvent donc être employés seuls ou en mélange avec de l'eau. Avantageusement, le solvant organique peut être choisi parmi l'éthanol, l'isopropanol, l'alcool benzylique et leurs mélanges.

La composition selon l'invention peut en outre comprendre au moins un ingrédient cosmétique usuel, différent des composés de l'invention, et notamment choisi parmi les propulseurs; les huiles végétales, minérales, animales ou de synthèse; les corps gras solides et notamment les cires, les esters en C8-C40, les acides en C8-C40; les alcools en C8-C40; les tensioactifs non ioniques, les tensioactifs cationiques, les tensioactifs anioniques, les tensioactifs amphotères, les tensioactifs zwittérioniques; les filtres solaires; les agents hydratants; les agents antipelliculaires; les agents antioxydants; les agents chélatants; les agents réducteurs; les bases d'oxydation, les coupleurs, les agents oxydants, les colorants directs; les agents défrisants tels que les thiols et les hydroxydes alcalins; les agents nacrants et opacifiants; les agents plastifiants ou de coalescence; les hydroxyacides; les pigments; les charges; les silicones et en particulier les polydiméthylsiloxanes (PDMS); les polymères conditionneurs ou coiffants; les parfums; les agents d'alcalinisation ou d'acidification; les silanes; les agents de réticulation tels que les polyphénols, les aldéhydes, la DHA. La composition peut bien évidemment comprendre plusieurs ingrédients cosmétiques figurant dans la liste ci-dessus.

Selon leur nature et la destination de la composition, les ingrédients cosmétiques usuels peuvent être présents en des quantités usuelles, aisément déterminables par l'homme du métier, et qui peuvent être comprises, de manière générale, pour chaque ingrédient, entre 0,01 à 80% en poids.

Les huiles peuvent être préférentiellement présentes à raison de 0,01 à 80% en poids, notamment 0,02 à 40% en poids, voire 0,5 à 20% en poids, par rapport au poids total de la composition. Elles peuvent être carbonées. On peut notamment citer les huiles végétales, animales ou minérales, hydrogénées ou non, les huiles synthétiques hydrocarbonées, cycliques ou aliphatiques, linéaires ou ramifiées, saturées ou insaturées, telles que par exemple les polyalpha-oléfines, en particulier les polydécènes et polyisobutènes; les alcools gras liquides tels que l'alcool isostéarylique, l'octyldodécanol, le 2-butyloctanol, le 2-hexyldécanol, le 2-undécylpentadécanol, l'alcool oléique ou l'alcool linoléique; les esters liquides comme le myristate d'isopropyle ou le palmitate d'isopropyle. On peut aussi citer les huiles de silicone, volatiles ou non, organomodifiées ou non, hydrosolubles ou non; les huiles fluorées ou perfluorées; ainsi que leurs mélanges.
La composition peut aussi comprendre un ou plusieurs corps gras solides, et en particulier un ou plusieurs alcools gras, esters gras et/ou acides gras, différents des huiles ci-dessus, ayant 8 à 40 atomes de carbone; ces corps gras solides peuvent préférentiellement être présents à raison de 0,01 à 30% en poids, notamment 0,1 à 20% en poids par rapport au poids total de la composition. On peut notamment citer les alcools gras à chaînes linéaires en C12-C32, notamment en C12-C26, et en particulier l'alcool cétylique, l'alcool stéarylique, l'alcool cétyls-téarylique, l'alcool béhénylique. On peut citer également les acides gras à chaînes linéaires ou ramifiées en C16-C40, et notamment l'acide méthyl-18 eicosanoïque, les acides d'huile de coprah ou d'huile de coprah hydrogénée; l'acide stéarique, l'acide laurique, l'acide palmitique et l'acide oléique, l'acide béhénique, et leurs mélanges. De préférence, les acides gras sont non salifiés. On peut encore citer les esters gras à chaînes linéaires, comportant au total entre 8 et 40 atomes de carbone, tels que les myristates, palmitates et stéarates de myristyle, de cétyle, de stéaryle, seuls ou en mélange.
L'homme de métier veillera à choisir les ingrédients entrant dans la composition, ainsi que leurs quantités, de manière à ce qu'ils ne nuisent pas aux propriétés des compositions de la présente invention.

Le pH de la composition, si elle est aqueuse, peut être acide, neutre ou alcalin. De préférence, la composition présente un pH compris entre 2 et 11, notamment 3 à 9, voire 3 à 7.

La composition cosmétique selon l'invention peut se présenter sous la forme d'un produit de soin, de nettoyage et/ou de maquillage de la peau du corps ou du visage, des lèvres, des sourcils, des cils, des ongles et des cheveux, d'un produit solaire ou autobronzant, d'un produit d'hygiène corporelle, d'un produit capillaire, notamment de soin, de nettoyage, de coiffage, de mise en forme, de coloration des cheveux.

Elle trouve notamment une application particulièrement intéressante dans le domaine capillaire, notamment pour le maintien de la coiffure ou la mise en forme des cheveux, ou encore le soin, le traitement cosmétique ou le nettoyage des cheveux. Les compositions capillaires sont de préférence des shampooings, des après-shampooings, des gels de coiffage ou de soin, des lotions ou crèmes de soin, des conditionneurs, des masques, des sérums, des lotions de mise en plis, des lotions pour le brushing, des compositions de fixation et de coiffage telles que les laques ou spray; de lotion restructurante pour cheveux; de lotion ou gel anti-chute, de shampoing antiparasitaire, de lotion ou shampoing antipelliculaire, de shampoing traitant antiséborrhéique. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

La composition selon l'invention trouve une application particulièrement intéressante pour le soin, le traitement et/ou le nettoyage des cheveux et/ou du cuir chevelu, par exemple en shampooing, en après shampooing, en post-traitement d'un shampoing et/ou d'un après-shampoing; ou bien entre deux shampoings.
On peut ainsi par exemple appliquer la composition selon l'invention sur le cuir chevelu et/ou les cheveux, directement après un shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut également appliquer la composition selon l'invention sur le cuir chevelu et/ou les cheveux directement après un après-shampooing, sur cheveux humides ou sur cheveux secs, avec ou sans temps de pause, avec ou sans chaleur, ladite application étant suivie ou pas d'un rinçage et d'un séchage, soit à température ambiante soit avec un sèche-cheveu ou encore avec un fer à lisser (250°C) par exemple.
On peut encore appliquer la composition selon l'invention sur le cuir chevelu et/ou les cheveux entre deux shampooings, sur cheveux humides ou sur cheveux secs.

La composition cosmétique peut être rincée ou non après avoir été appliquée sur les matières kératiniques (cheveux et/ou cuir chevelu).

L'invention a également pour objet un procédé de traitement capillaire, pour le soin, le traitement cosmétique et/ou le nettoyage des cheveux et/ou du cuir chevelu, et en particulier permettant de retarder voire d'éviter le regraissage des cheveux et/ou du cuir chevelu, et ainsi d'espacer les shampoings, comprenant l'application sur lesdites matières d'une composition cosmétique selon l'invention.

L'invention est illustrée plus en détails dans les exemples suivants.

### Exemple 1

On prépare la composition de soin capillaire suivante (% en poids) :

| | Composition |
|---|---|
| SILICA SILYLATE (nom INCI) | 0,5% |
| Polymère acrylique (Carbopol 980) | 0,3% |
| Eau | Qsp 100% |

Le SILICA SILYLATE employé est le produit commercialisé sous le nom DOW CORNING VM-2270 AEROGEL FINE PARTICLES par Dow Corning.

La composition se présente sous forme d'une dispersion de pH égal à 3,5 et est facile à appliquer sur les cheveux et le cuir chevelu; elle permet de réduire le regraissage des cheveux.

## Revendications

1. Procédé de traitement capillaire, pour le soin, le traitement cosmétique et/ou le nettoyage des cheveux et/ou du cuir chevelu, en particulier permettant de retarder voire d'éviter le regraissage des cheveux et/ou du cuir chevelu, comprenant l'application d'une composition cosmétique comprenant des particules d'aérogel de silice hydrophobe et au moins un polymère épaississant acrylique choisi parmi les homopolymères d'acide (méth)acrylamido alkyl(C1-C4) sulfonique, éventuellement réticulés.

2. Procédé selon la revendication 1, dans lequel les particules d'aérogel de silice hydrophobe présentent une surface spécifique par unité de masse (SM) allant de 500 à 1500 m²/g, de préférence de 600 à 1200 m²/g et mieux de 600 à 800 m²/g,

3. Procédé selon l'une quelconque des revendications précédentes, dans lequel les particules d'aérogel de silice hydrophobe présentent une capacité d'absorption d'huile mesurée au WET POINT allant de 5 à 18 ml/g de particules, de préférence de 6 à 15 ml/g et mieux de 8 à 12 ml/g.

4. Procédé selon l'une quelconque des revendications précédentes, dans lequel les particules d'aérogel de silice hydrophobe présentent une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm.

5. Procédé selon l'une quelconque des revendications précédentes, dans lequel les particules d'aérogel de silice hydrophobe présentent une densité tassée p allant de 0,04 g/cm³ à 0,10 g/cm³, de préférence de 0,05 g/cm³ à 0,08 g/cm³.

6. Procédé selon l'une quelconque des revendications précédentes, dans lequel les particules d'aérogel de silice hydrophobe présentent une surface spécifique par unité de volume (SV) allant de 5 à 60 m²/cm³, de préférence de 10 à 50 m²/cm³ et mieux de 15 à 40 m²/cm³.

7. Procédé selon l'une quelconque des revendications précédentes, dans lequel les particules d'aérogel de silice hydrophobe sont des particules d'aérogel de silice silylée et notamment des particules d'aérogels de silice hydrophobe modifiée en surface par groupements triméthylsilyles.

8. Procédé selon l'une quelconque des revendications précédentes, dans lequel les particules d'aérogel de silice hydrophobe sont présentes en une quantité comprise entre 0,01 et 20% en poids, de préférence entre 0,05 et 10% en poids, préférentiellement entre 0,1 et 5% en poids par rapport au poids total de la composition.

9. Procédé selon l'une quelconque des revendications précédentes, dans lequel le ou les polymères épaississants acryliques sont présents en une quantité comprise entre 0,01 et 2% en poids, notamment 0,1 à 0,5% en poids, par rapport au poids total de la composition.

10. Procédé selon l'une quelconque des revendications précédentes, dans lequel la composition comprend de l'eau à une concentration allant de 5 à 98% en poids, notamment de 20 à 95% en poids, mieux de 50 à 90% en poids, par rapport au poids total de la composition.
